Europäisches Patentamt

European Patent Office

Office européen des brevets

(19)

(11) Publication number : **0 556 962 A1**

## (12) EUROPEAN PATENT APPLICATION

(21) Application number : **93300585.2**

(22) Date of filing : **27.01.93**

(51) Int. Cl.⁵ : **C07K 7/08, C07K 15/00, A61K 37/43**

(30) Priority : **11.02.92 HU 40692**

(43) Date of publication of application :
**25.08.93 Bulletin 93/34**

(84) Designated Contracting States :
**AT BE CH DE DK ES FR GB GR IT LI LU NL SE**

(71) Applicant : **RICHTER GEDEON VEGYESZETI GYAR R.T.**
**Gyömröi ut 19-21**
**H-1475 Budapest (HU)**

(72) Inventor : **Orosz, Antal**
**Erdoalja ut 110**
**H-1037 Budapest (HU)**
Inventor : **Nyeki, Olga**
**Szlacsanyi Ferenc u. 38**
**H-1151 Budapest (HU)**

Inventor : **Schon, Istvan**
**Raskai Lea u. 86**
**H-1142 Budapest (HU)**
Inventor : **Kisfaludy, Lajos**
**deceased (HU)**
Inventor : **Schrett, Janos**
**Harmatcsepp u. 41**
**H-1028 Budapest (HU)**
Inventor : **Bartha, Laszlo**
**Csango u. 22/A**
**H-1134 Budapest (HU)**
Inventor : **Nagy, Jozsef**
**Baross u. 54**
**H-1181 Budapest (HU)**
Inventor : **Rill, Attila**
**Varosmajor u. 31/B**
**H-1122 Budapest (HU)**
Inventor : **Balogh, Gabor**
**Csontvary K.T. u. 18**
**H-1181 Budapest (HU)**

(74) Representative : **Pett, Christopher Phineas**
**Frank B.Dehn & Co. Imperial House 15-19**
**Kingsway**
**London WC2B 6UZ (GB)**

(54) **Small peptide and pseudopeptide amides inhibiting the proliferation of small-cell and epithelial-cell lung cancer cells.**

(57) The invention relates to novel peptide and pseudo-peptide amides of the formulae (1) to (6), wherein

$$\underline{p}\text{-HOPA-D-Trp-Phe-D-Trp-Leu}\ \psi(CH_2NH)Leu\text{-}NH_2 \qquad (1)$$

$$D\text{-MePhe-D-Trp-Phe-D-Trp-Leu}\ \psi(CH_2NH)Leu\text{-}NH_2 \qquad (2)$$

$$D\text{-MePhe-D-Trp-Phe-D-Trp-Leu-MPA} \qquad (3)$$

$$D\text{-Tyr-D-Trp-Phe-D-Trp-Leu}\ \psi(CH_2NH)Leu\text{-}NH_2 \qquad (4)$$

$$D\text{-Tyr(Et)-D-Trp-Phe-D-Trp-Leu}\ \psi(CH_2NH)Leu\text{-}NH_2 \qquad (5)$$

$$D\text{-MePhe-D-Trp-Tyr-D-Trp-Leu}\ \psi(CH_2NH)Leu\text{-}NH_2 \qquad (6)$$

wherein
p-HOPA means p-hydroxyphenylacetic acid,
D-MePhe stands for D-N-methylphenylalanine,
MPA represents 2-amino-3-methylpentane and
$\psi(CH_2NH)$ is a methyleneamino group being present instead of a peptide bond,

as well as their pharmaceutically acceptable acid addition salts.

The invention further relates to pharmaceutical compositions containing these compounds as well as processes for preparing the compounds of formulae (1) to (6).

The compounds according to the invention are useful for inhibiting the proliferation of cells of the small-cell and epithelial-cell lung cancer.

This invention relates to peptides and pseudopeptides, processes for their preparation, and pharmaceutical compositions containing them.

It is known [Nature 316, 823 (1985)] that both bombesin (BN) occurring in the amphibia as well as the gastrin-releasing peptide (GRP), which is structurally closely related to bombesin and occurs in mammals, act as autocrine growth factors in small-cell lung cancer (SCLC). The formula of bombesin is

$$\text{Glp-Gln-Arg-Leu-Gly-Asn-Gln-Trp-Ala-Val-Gly-His-Leu-Met-NH}_2,$$

whereas the fragment 14 to 27 of GRP is represented by the formula

$$\text{Met-Tyr-Pro-Arg-Gly-Asn-His-Trp-Ala-Val-Gly-His-Leu-Met-NH}_2.$$

It is also known [Am. J. Physiol. 252, 6439 (1987); Regulatory Peptides 19, 105 (1987); as well as Eur. J. Pharmacol. 190, 31 (1990)] that structural analogues of BN or GRP (BN antagonists) inhibit the binding of [$^{125}$I-Tyr$^4$]-bombesin to BN receptors being present on Swiss 3T3 cells and prevent the proliferation of the small-cell lung cancer cell line; said proliferation can be measured by the incorporation of $^3$H-thymidine.

It has been observed [Proc. Natl. Acad. Sci. USA 82, 7616 (1985); Br. J. Cancer 57, 579 (1988)] that the peptide of the formula D-Arg-D-Pro-Lys-Pro-Gln-Gln-D-Trp-Phe-D-Trp-Leu-Leu-NH$_2$ (Spantide), an antagonist against "Substance P" (SP), acts as a BN antagonist on Swiss 3T3 mouse embryonal fibroblasts and inhibits the proliferation of the cells of the small-cell lung cancer.

Furthermore, it has been described [Biochem. Biophys. Res. Commun. 156, 323 (1988)] that the N-acylated pentapeptide of the formula

$$\text{HOPA-D-Trp-Phe-D-Trp-Leu-Leu-NH}_2 \qquad\qquad \text{(I)}$$

is a relatively strong SP antagonist (with a pA$_2$ value of 5.73 on guinea pig ileum) inhibiting the smooth muscle-contracting effect of SP.

Our investigations concerning the C-terminal pentapeptide analogues of Spantide have shown the low molecular SP antagonist of the formula (I) to inhibit the binding of the labelled [$^{125}$I-Tyr$^4$]-bombesin to Swiss 3T3 cells and prevent the proliferation of NCI-H69 small-cell lung cancer cell line possessing no BN receptors.

Thus, one aim of the present invention is to prepare analogues not inhibiting the binding of [$^{125}$I-Tyr4]-BN to Swiss 3T3 cells, i.e. analogues which are therefore not BN antagonists but exert a strong inhibitory action on the proliferation of the cells of the small-cell and epithelial-cell lung cancers. Such compounds might be very useful in the therapy of lung cancer.

We have found that analogues having formulae (1) to (6) below, containing a modified, mainly reduced peptide bond, show such an effect.

Thus, according to one aspect of the present invention, we provide peptide and pseudopeptide amides of the formulae (1) to (6),

$$\underline{p}\text{-HOPA-D-Trp-Phe-D-Trp-Leu}\ \psi\text{(CH}_2\text{NH)Leu-NH}_2 \qquad\qquad \text{(1)}$$

$$\text{D-MePhe-D-Trp-Phe-D-Trp-Leu}\ \psi\text{(CH}_2\text{NH)Leu-NH}_2 \qquad\qquad \text{(2)}$$

$$\text{D-MePhe-D-Trp-Phe-D-Trp-Leu-MPA} \qquad\qquad \text{(3)}$$

$$\text{D-Tyr-D-Trp-Phe-D-Trp-Leu}\ \psi\text{(CH}_2\text{NH)Leu-NH}_2 \qquad\qquad \text{(4)}$$

$$\text{D-Tyr(Et)-D-Trp-Phe-D-Trp-Leu}\ \psi\text{(CH}_2\text{NH)Leu-NH}_2 \qquad\qquad \text{(5)}$$

$$\text{D-MePhe-D-Trp-Tyr-D-Trp-Leu}\ \psi\text{(CH}_2\text{NH)Leu-NH}_2 \qquad\qquad \text{(6)}$$

wherein
p-HOPA, means p-hydroxyphenylacetic acid,
D-MePhe stands for D-N-methylphenylalanine,
MPA represents 2-amino-3-methylpentane and
$\psi$(CH$_2$NH) is a methyleneamino group being present instead of a peptide bond,
as well as their pharmaceutically acceptable acid addition salts.

The compounds of formulae (1) - (6) according to the invention possess valuable pharmacological activity.

More particularly, they inhibit the proliferation of the cells of the small-cell and epithalial cell lung cancers.

Abbreviations used in the description are as follows:

FCS      foetal calf serum
D-MEM      Dulbecco's minimal essential medium
BSA      bovine serum albumin
HEPES      N-(2-hydroxyethyl)piperazine-N'-(2-ethanesulfonic acid)
PBS      phosphate-buffered saline
SDS      sodium dodecyl sulfate
TCA      trichloroacetic acid

Other abbreviations are in agreement with those described in "Nomenclature and Symbolism for Amino Acids and Peptides, Recommendations 1983" of the IUPAC-IUB Joint Commission on Biochemical Nomenclature (JCBN) [Biochem. J. 219, 345 (1984)].

The compounds of formulae (1) to (6) and their acid addition salts can be prepared in such a way that, by using Leu $\psi(CH_2NH)$Leu-$NH_2$ or Leu-MPA as starting substances, the peptide of formula (1), or the derivatives of peptides of formulae (2) to (6) containing N-terminal benzyloxycarbonyl or tert-butoxycarbonyl protective group are built up. Preferably this is achieved by the successive use of the steps of reactive ester coupling and deprotection of the $\alpha$-$NH_2$ group. The protective group may be removed by catalytic hydrogenation or acidolysis and, if desired, the product obtained is transformed into a pharmaceutically acceptable acid addition salt or the free base is liberated from such a salt.

In the case of the compound of formula (1), the N-terminal p-HOPA may be incorporated without any protective group into the molecule, to obtain the final product by building up the peptide chain.

The methyleneamino (reduced peptide) bond of the pseudopeptide Leu- $\psi(CH_2NH)$Leu-$NH_2$, used as starting substance, can be formed by reductive alkylation performed in a known manner [see e.g. Nature 299, 555 (1982)]. Thus, Leu-$NH_2$ hydrochloride is alkylated with Z-Leu -H (Z-Leu-aldehyde) and the Schiff's base obtained is reduced in situ by sodium cyanoborohydride. The N-terminal benzyloxycarbonyl protective group of the protected pseudodipeptide Z-Leu $\psi(CH_2NH)$Leu-$NH_2$ is removed by catalytical hydrogenation.

Leu-MPA may be prepared as follows. In the first step, 2-amino-3-methylpentane is acylated by a mixed anhydride formed from Z-Leu-OH and pivaloyl chloride, then the benzyloxycarbonyl protective group is removed by catalytical hydrogenation and the mixture of Leu-MPA isomers is separated by chromatography on a silica gel column. Thereafter, the Leu-MPA isomer described in the experimental part is used.

After accomplishment of the synthesis, the crude peptide amides obtained may be purified e.g. by chromatography on a silica gel column.

The biological investigation of the new compounds of the invention was carried out in four tests. The BN-antagonizing effect of the compounds in question was determined in Test 1, based on measuring the displacement of the labelled [$^{125}$I-Tyr$^4$]-BN from the BN receptors being present on the Swiss 3T3 mouse fibroblast cells. Test 2 and Test 3 were used to study the inhibitory effect of the compounds of the invention on the proliferation and propagation of NCI-H69 small-cell lung cancer cells. The inhibitory effect of the compounds of the invention on non-small-cell lung cancer cells and epithelial-cell lung cancer cells was investigated in Test 4.

The reference compound (I) in Tables 1-4 below is listed at page 2, line 1 above.

**Test 1**

**Study of the binding of target compounds to BN receptors on the Swiss 3T3 mouse fibroblast cell line**

Swiss 3T3 mouse fibroblast cells were grown on tissue-cultivating plates containing 24 holes in a D-MEM culture medium containing FCS of 10 % until a confluent and resting state ($3 \times 10^5$ cell/hole). The cells were washed 3 times with 0.5 ml of D-MEM of pH 7.4 (containing 0.2 % of BSA and 24 mmol of HEPES, and cooled to 4 °C), i.e. incubation mixture, each. Subsequently, the cells were incubated in a final volume of 250 $\mu$l at 4 °C for 3 hours with an incubation mixture containing [$^{125}$I-Tyr$^4$]-BN in a constant concentration (1 nmol) and the compound to be tested in an amount of 1.18 to 75 $\mu$mol. At the end of the reaction the cells were washed twice with the incubation mixture and then 3 times with PBS (pH = 7.4).

The washings were carried out with 750 $\mu$l volumes of the ice-cold liquids each. The cells were solubilized by adding 750 $\mu$l of 0.5 M sodium hydroxide solution, carried over into test-tubes by pipette and the radio-activity bound to the cells was measured. The grade of aspecific binding was determined in the presence of a high concentration ($10^{-6}$ mol/l) of cold [Tyr$^4$]-BN. The amount of the specifically bound, labelled hormone was expressed as the percentage of the maximum binding, i.e. the specific binding in the absence of the compounds to be tested, as shown in Table 1.

**Table 1**

| Displacement of [$^{125}$I-Tyr$^4$]-BN on the Swiss 3T3 mouse fibroblast cell line | | | | | | |
|---|---|---|---|---|---|---|
| Concentration | Displacement as percentage of control net binding | | | | | |
| | Compound | | | | | |
| [μmol/l] | (I) | (1) | (2) | (3) | (4) | (5) |
| 0 | 100 | 100 | 100 | 100 | 100 | 100 |
| 1.18 | 108 | 105 | - | 96 | 85 | 113 |
| 2.36 | 98 | 96 | 92 | 108 | 88 | 111 |
| 4.71 | 83 | 99 | 109 | 92 | 79 | 106 |
| 9.42 | 55 | 103 | 93 | 103 | 72 | 117 |
| 18.8 | 33 | 106 | 99 | 108 | 61 | 116 |
| 37.6 | 18 | 107 | 94 | 113 | 52 | 95 |
| 75 | 23 | 105 | 91 | 97 | 45 | 81 |

The data of Table 1 show that the BN-antagonizing effect characteristic of SP antagonists is avoided by incorporating the methyleneamino bond into SP antagonists [compounds of formulae (1), (2), (4) and (5)] or by substituting an aliphatic amine for the C-terminal amino acid of the SP antagonists [compound (3)].

**Test 2**

**Investigation of the inhibitory effect of target compounds on the proliferation of NCI-H69 small-cell lung cancer cell line possessing no BN receptors by measuring the incorporation of $^3$H-thymidine**

The cells grown in suspension, i.e. in RPMI-1640 culture medium (DIFCO, U.S.A.) supplemented with FCS of 10 10%, were collected by centrifuging at 4 °C with 1200 rpm. After decanting the supernatant, the cells were suspended in RPMI-1640 HITES medium (containing $1 \times 10^{-8}$ mol/l of hydrocortisone, 5 mg/l of insulin, 10 mg/l of transferrin, $1 \times 10^{-8}$ mol/l of estradiol and $3 \times 10^{-8}$ mol/l of sodium selenite). The cell number was adjusted to $5 \times 10^5$ cell/ml; 90 μl of this cell suspension were added into each hole of a tissue-cultivating plate containing 96 holes. 10 μl of solutions, each containing the target compounds in various concentrations, were added to the cells. The cells were grown at 37 °C under air containing 5 % by volume of carbon dioxide for 72 hours, then 37 kBq (1 μCi) of $^3$H-thymidine, dissolved in 10 μl of culture medium, were added into the holes and the incubation was continued for an additional 24 hours. Subsequently, the cells were solubilized in 10 μl of 20 % SDS solution. 25 μl of solution from each hole were dropped onto Whatman 3 filter paper. After drying, the filter paper was washed 3 times with 5 % aqueous cold trichloroacetic acid solution, and then with 96 % ethanol in order to dehydrate it and to remove the traces of trichloroacetic acid.

After drying, the filter paper pieces were placed in scintillation tubes and 5 ml of scintillation cocktail [a mixture containing 1000 ml of toluene, 400 ml of abs. ethanol, 10 ml of dioxane, 6 g of 2,5-diphenyloxazole and 0.15 g of 1,4-bis(5-phenyl-2-oxazolyl)benzene] were added to each. The activity of the samples was measured by using a LKB Wallac 1211 beta-counter. The data were expressed as the percentage of control, where the $^3$H-thymidine uptake of NCI-H69 cells not treated with the compounds was considered to be 100 % (control) (see Table 2).

### Table 2

| Effect of the target compounds on the $^3$H-thymidine uptake of NCI-H69 small-cell lung cancer cell line | | | | | | | |
|---|---|---|---|---|---|---|---|
| Concentration | Uptake as percentage of control | | | | | | |
| | Compound | | | | | | |
| [μmol/l] | (I) | (1) | (2) | (3) | (4) | (5) | (6) |
| 0 | 100 | 100 | 100 | 100 | 100 | 100 | 100 |
| 0.75 | 92 | - | 75 | 115 | 101 | 105 | - |
| 1.56 | 77 | - | 48 | 104 | 76 | 155 | 82 |
| 3.125 | 104 | 77 | 51 | 49 | 88 | 137 | 65 |
| 6.25 | 66 | 34 | 20 | 34 | 59 | 67 | 63 |
| 12.5 | 63 | 44 | 8 | 18 | 51 | 17 | 58 |
| 25 | 6 | 39 | 4 | 31 | 9 | 9 | 49 |
| 50 | 4 | 30 | 4 | 7 | 5 | 7 | 44 |
| 100 | 3 | 2 | 4 | - | - | - | |
| 200 | 1.7 | 1.3 | 1.7 | - | - | - | |

**Test 3**

**Investigation of the inhibitory effect of target compounds on the propagation of NCI-H69 small-cell lung cancer cells**

NCI-H69 cells grown in suspension, i.e. in RPMI-1640 culture medium supplemented with FCS of 10 %, were collected by centrifuging at 4 °C with 1500 rpm. After decanting the supernatant, the cells were suspended in a fresh medium and the cell number was adjusted to 1 x $10^5$ cell/ml. 5 ml of cell suspension each were applied into tissue-cultivating vessels of 25 cm² size. The compounds to be tested were used in a final concentration of 10 and 50 μmol/l, respectively. The cell number was determined from 2x200 μl of cell suspension in each defined time interval. The volume of sample taken out was replaced with a medium containing the target compound in a concentration of 10 or 50 μmol/l, respectively. The cell resultant dilution was corrected by calculation. The data were expressed as percentage of the starting cell number (1x$10^5$ cell/ml was considered to be 100 %). The change in the number of cells not treated with the compounds was also indicated as control (see Table 3).

## Table 3

### Effect of the target compounds on the propagation of NCI-H69 small-cell lung cancer cell line

| Concent-ration [$\mu$mol/l] | Cell number as percentage of the starting cell number | | | | | | | | |
|---|---|---|---|---|---|---|---|---|---|
| | Compound | | | | | | | | |
| | (C) | (I) | | (2) | | (3) | | (4) | (5) |
| | 0 | 10 | 50 | 10 | 50 | 10 | 50 | 50 | 50 |
| t   days | | | | | | | | | |
| 0 | 100 | 100 | 100 | 100 | 100 | 100 | 100 | 100 | 100 |
| 3 | 157 | 213 | 90 | 130 | 25 | 110 | 65 | 57 | 60 |
| 5 | 164 | – | – | 110 | 47 | 150 | 93 | – | 73 |
| 7 | 175 | 233 | 120 | 140 | 20 | 190 | 107 | 80 | 122 |
| 10 | 204 | 182 | 75 | 100 | 10 | 150 | 97 | 87 | 105 |
| 12 | 257 | – | – | 110 | 20 | 170 | 120 | 74 | 120 |

Note: C = control

It can be seen from the data of Tables 2 and 3 that the target compounds retained the inhibitory effect on the maturation and propagation of NCI-H69 small-cell lung cancer cells, which is characteristic of SP antagonists; the inhibiting effect of the compound of formula (2) is even stronger than that of the reference substance of the formula (I).

**Test 4**

**Examination of the effect of target compounds on the proliferation of BR-MES-1 epithelial-cell lung cancer cell line by determining the incorporation of $^3$H-thymidine**

The cells cultivated in a monolayer culture in D-MEM medium supplemented with FCS of 10 % were suspended by treatment with trypsin of 0.25 % and the cell concentration was adjusted to 5 x 10$^5$ cell/ml by adding fresh culture medium.

100 $\mu$l of cell suspension were applied into each of 96 holes of a tissue-cultivating plate. After 48-hour incubation at 37 °C under air containing 5 % by volume of carbon dioxide, the culture medium on the adhered cells was exchanged for 90 $\mu$l of D-MEM-HITES-0.1 % BSA. 10 $\mu$l of solutions each containing the target compounds in various concentrations were added to the cells and after 24 hours, 37 kBq (1 $\mu$Ci) of $^3$H-thymidine were added into each hole, then the incubation was continued for an additional 24 hours.

The samples were taken and measured as described above for Test 2.

The data were expressed as percentage of the control. The $^3$H-thymidine uptake of SK-MES-1 cells not treated with the compounds was considered to be 100 % (control).

## Table 4

### Effect on the $^3$H-thymidine uptake of SK-MES-1 epithalial-cell lung cancer cell line

| Concentration [μmol/l] | Thymidine uptake as percentage of control | | | | | |
|---|---|---|---|---|---|---|
| | Compound | | | | | |
| | (I) | (2) | (3) | (4) | (5) | (6) |
| 0 | 100 | 100 | 100 | 100 | 100 | 100 |
| 6.25 | 188 | 33 | 109 | 81 | 44 | 63 |

## Table 4 (contd.)

| Concentration [μmol/l] | Thymidine uptake as percentage of control | | | | | |
|---|---|---|---|---|---|---|
| | Compound | | | | | |
| | (I) | (2) | (3) | (4) | (5) | (6) |
| 12.5 | 179 | 25 | 92 | 98 | 6 | 58 |
| 25 | 187 | 17 | 65 | 64 | 10 | 49 |
| 50 | 67 | 1 | 1 | 6 | 1 | 44 |

The data of Table 4 indicate that the spectrum of inhibitory effects of the compounds according to the invention on the proliferation of lung cancer cells is broader than those of the known SP antagonists [see the reference substance of formula (I)]. The compounds of the invention also prevent the proliferation of SK-MES-1 epithelial-cell lung cancer cell line.

According to a further aspect of the present invention there are provided pharmaceutical compositions comprising as active ingredient at least one peptide or pseudopeptide amide of the formulae (1) - (6) of the present invention or an acid addition salt thereof in admixture with a pharmaceutically acceptable carrier,diluent or excipient. The pharmaceutical compositions of the present invention can be used in therapy for inhibiting the proliferation of cells of the small-cell and epithelial-cell lung cancer.

The peptide and pseudopeptide amides of the formulae (1) - (6) and salts thereof may be formulated in forms generally used in therapy and by methods of manufacture known per se. The pharmaceutical compositions of the present invention may be formulated in solid, liquid or semiliquid forms and may contain one or more of generally used conventional carriers, diluents, fillers, auxiliary agents (e.g. stabilizing agents, salts for modifying the osmotic pressure), agents for adjusting the pH value and further additives.

The solid pharmaceutical compositions may be e.g. tablets, dragées, capsules, wafers or powder ampoules useful in the preparation of injections. The liquid compositions may be e.g. injections, infusions, spoonfuls, wet packs and drops. The semiliquid compositions may be e.g. creams, ointments, balms, shaking mixtures or suppositories.

The pharmaceutical compositions of the present invention are administered in an amount which contains sufficient active ingredient to exhibit the desired effect.The said dose depends on the type and severity of the disease, the body weight of the patient and his (or her) sensitivity against the active ingredient, the mode of application, the daily number of treatments, etc. The dose to be applied can be safely determined by the physician based on all circumstances of the given case.

In order to enable simple administration, the active ingredient is preferably provided in the form of dosage units which contain the active ingredient in the amount to be administered or a small multiple or part (e.g. half, one-third, one-fourth part) thereof. Such dosage units are e.g. the tablets which may be provided by groove(s) in order to simplify the division of a tablet into two or four parts.

To ensure the hypogastric absorption of the active ingredient the tablets may be provided with a coating not soluble in acidic medium; i.e the tablets can be rendered enterosolvent. Similar effect can be achieved by encapsulating the active ingredient.

The pharmaceutical compositions of the present invention may generally contain from about 1 mg to about 100 mg of the active ingredient per dosage unit. The above values are naturally of a mere illustrative character and the actual active ingredient content can be below or above the said limits if desired.

The invention is illustrated in detail by the aid of the following non-limiting Examples.

Melting points were determined on a Tottoli's device (Büchi, Switzerland).

Thin layer chromatography (TLC) examinations were carried out on prefabricated silica gel adsorbent layers (DC-Fertigplatten, Merck) by using the following solvent systems:

1. Ethyl acetate:stock solution = 19:1 (sign[1])
2. Ethyl acetate:stock solution = 4:1 (sign[2])
3. Ethyl acetate:stock solution = 39:1 (sign[3])
4. Ethyl acetate:methanol:n-hexane = 6:1:3 (sign[4]).

The stock solution was a 20:6:11 mixture of pyridine/acetic acid/water. The above quotients are volume ratios.

The chromatograms were detected by ninhydrin as well as by using KI/o-tolidine reagent after chlorination.

High pressure liquid chromatography (HPLC) examinations were carried out on a BST Nucleosil 300 C18 5 μm column. A Gilson 305 detector connected with a Gilson pump was used in these examinations. As eluent a 2:3 mixture of acetonitrile/water containing 0.1 % of trifluoroacetic acid was used with a flow rate of 1.2 ml/min.

When investigated by this system, the purity of the compounds of the invention was found to be at least 95 %.

For amino acid analyses, the samples were parallel hydrolyzed in 6 mol/l hydrochloric acid and mercaptosulfonic acid at 110 °C under nitrogen for 24 hours. The hydrolysates were examined in a Biotronik LC 5000 amino acid analyzer. In addition to the common amino acids, MePhe and Leu $\psi$(CH$_2$NH)Leu pseudodipeptide were also detected in the hydrolysate.

NMR spectra of the new compounds were taken on a Varian VXR-300 type NMR spectrometer at room temperature in deuterated dimethylsulfoxide (DMSO-d$_6$) solution, by using tetramethylsilane (TMS) as internal standard. The signal of the CH$_2$ moiety of the methyleneamino group appeared in the $^1$H-NMR spectrum as a multiplet close to 2.4 ppm; whereas the signal of the CH$_2$ moiety showing a triplet multiplicity was found close to 52 ppm in the $^{13}$C-NMR spectrum and simultaneously, the signal of the carbon atom in the C=O moiety of the peptide bond was absent.

**Example 1**

**Preparation of Z-MePhe-D-Trp-Phe-D-Trp-Leu $\psi$(CH$_2$NH)Leu-NH$_2$**

**(1) (a) Z-Leu $\psi$(CH$_2$NH)Leu-NH$_2$**

After suspending 3.6 g (100 mmol) of lithium aluminum hydride in 30 ml of tetrahydrofuran (THF) cooled to 0 °C, the solution of 14.6 g (200 mmol) of diethylamine in 50 ml of n-hexane is dropwise added thereto at 0 °C. After stirring the suspension at 0 °C for 10 minutes, 21.3 g (76 mmol) of Z-Leu-OCH$_3$ dissolved in 120 ml of n-hexane are portionwise added. After stirring at 0 °C for 90 minutes, the reaction mixture is diluted with 70 ml of ethyl acetate and acidified to pH 2 by adding 6 mol/l hydrochloric acid solution under strong cooling. After separation of the two phases, the aqueous layer is extracted with ethyl acetate, then the combined organic phase is washed with cold water. The ethyl acetate solution is dried over anhydrous sodium sulfate and evaporated.

The Z-Leu-H aldehyde obtained (18 g, 69 mmol) is dissolved in 300 ml of a 99:1 mixture of methanol/acetic acid cooled to 0 °C and 8 g (50 mmol) of Leu-NH$_2$.HCl are added. To the solution obtained 4.8 g (80 mmol) of sodium cyanoborohydride dissolved in 100 ml of anhydrous tetrahydrofuran are dropwise added at 0 °C. The reaction mixture is stirred at 0 °C for 30 minutes and then at room temperature for 2 hours.

After evaporating the solvent, the residue is dissolved in 300 ml of ethyl acetate, washed twice with 100 ml of 5 % sodium hydrogen carbonate solution each and twice with water. After drying, the solution is evaporated and the residue is solidified by adding 50 ml of n-hexane. The crude product is recrystallized from 20 ml of ethyl acetate to obtain 7 g (38 % yield calculated for Leu-NH$_2$·HCl) of Z-Leu $\psi$(CH$_2$NH)Leu-NH$_2$, m.p.: 108-110 °C.

**(1)(b) H-Leu $\psi$(CH$_2$NH)Leu-NH$_2$·2HCl**

To a solution containing 5.0 g (13.7 mmol) of Z-Leu $\psi$(CH$_2$NH)Leu-NH$_2$ in 100 ml of methanol, 5 ml of 5.3 mol/l hydrogen chloride solution in dioxane and then 0.5 g of 10 % palladium-on-carbon catalyst are added, then hydrogen is bubbled through the suspension.

The reaction becomes complete within 2 hours. After filtering off the catalyst, the filtrate is evaporated under reduced pressure, the residue is solidified by adding 50 ml of ether and the crude product obtained is recrystallized from an 1:5 mixture of ethanol and ether to give the aimed product in a yield of 3.4 g (82 %); $[\alpha]_D$ = +7.3° (c = 2, methanol), m.p.: 110 °C ( decomposition).

**(1)(c) Z-MePhe-D-Trp-Phe-D-Trp-Leu $\psi$(CH$_2$NH)Leu-NH$_2$**

1.12 ml (8 mmol) of triethylamine and 4.0 g (8.5 mmol) of Boc-D-Trp-OPfp (OPfp means pentafluorophenoxy group) are added to a solution of 2.4 g (8 mmol) of Leu (CH$_2$NH)Leu-NH$_2$·2HCl in 30 ml of dimethylformamide at 0 °C. After stirring the reaction mixture at room temperature for 90 minutes, meanwhile portionwise adding 1.5 ml of triethylamine, the reaction mixture is evaporated and the residue is dissolved in 100 ml of ethyl acetate. The solution is twice extracted with 30 ml of 1 mol/l hydrochloric acid each and then with 30 ml of water. The combined aqueous phase is neutralized by adding sodium carbonate and extracted twice with 30 ml of ethyl acetate each. After drying over anhydrous sodium sulfate, the ethyl acetate solution is evaporated. The protected tripeptide is solidified by adding ether to obtain a yield of 3.2 g (77 %), m.p.: 180 - 182 °C.

$R_f{}^1$ = 0.3 ($^1$ means solvent system 1, $^2$ means solvent system 2, etc.)

After dissolving 3.0 g (5.8 mmol) of Boc-D-Trp-Leu-$\psi$(CH$_2$NH)Leu-NH$_2$ in 15 ml of a 5.3 mol/l hydrogen chloride solution in dioxane, the solution is diluted with 100 ml of ether after 20 minutes. The precipitate is filtered off and washed with ether to give 2.8 g (98 %) of product, m.p.: 170 °C (decomposition); $R_f{}^2$ = 0.1.

After adding 1.4 ml (10 mmol) of triethylamine and 2.3 g (5.3 mmol) of Boc-Phe-OPfp to the solution of 2.45 g (5 mmol) of H-D-Trp-Leu $\psi$(CH$_2$NH)Leu-NH$_2$·2HCl in 30 ml of dimethylformamide, the reaction mixture is stirred at room temperature for 1 hour, then the dimethylformamide is distilled off and the residue is solidified by adding 50 ml of water. The precipitate obtained is filtered, washed with 50 ml of water and 30 ml of ethanol to obtain the pseudotetrapeptide Boc-Phe-D-Trp-Leu $\psi$(CH$_2$NH)Leu-NH$_2$ in a yield of 2.73 g (82 %), m.p.: 212-214 °C (decomposition); $R_f{}^2$ = 0.7.

2.5 g (3.7 mmol) of the pseudotetrapeptide Boc-Phe-D-Trp-Leu $\psi$(CH$_2$NH)Leu-NH$_2$ are dissolved in 30 ml of 5.3 mol/l hydrogen chloride solution in dioxane and after 30 minutes, the mixture is diluted with 100 ml of ether. After filtering and washing with 25 ml of ether, the precipitate is dissolved in 25 ml of dimethylformamide. To this solution, 1.3 ml of triethylamine and 1.9 g (4 mmol) of Boc-D-Trp-OPfp are added, the reaction mixture is stirred at room temperature overnight, then dimethylformamide is distilled off. The residue is dissolved in 50 ml of ethyl acetate and the solution is twice shaken with 20 ml of 1 mol/l hydrochloric acid solution each and then with 20 ml of water. The aqueous phases are extracted with 20 ml of ethyl acetate each. The combined organic solution is dried over anhydrous sodium sulfate and evaporated. The residue is solidified by adding ether to obtain the pseudopentapeptide in a yield of 2.83 g (90 %), m.p.: 133 °C (decomposition); $R_f{}^2$ = 0.7.

2.6 g (3 mmol) of Boc-D-Trp-Phe-D-Trp-Leu $\psi$-(CH$_2$NH)Leu-NH$_2$ are treated with 20 ml of 5.3 mol/l hydrogenchloride solution in dioxane, and after 30 minutes, the solution is diluted with 100 ml of ether. After filtering the precipitate and washing with ether, the free pseudopentapeptide dihydrochloride is obtained in a yield of 2.4 g (92 %), m.p.: 167 °C (with decomposition); $R_f{}^2$ = 0.15.

After dissolving 1.23 g (1.48 mmol) of H-D-Trp-Phe-D-Trp-Leu $\psi$(CH$_2$NH)Leu-NH$_2$·2HCl in 20 ml of dimethylformamide, 0.96 g (2 mmol) of Z-D-MePhe-OPfp and 0.7 ml (5 mmol) of triethylamine are added to the above solution. After stirring the reaction mixture at room temperature for 1 hour and evaporating, the residue is dissolved in 30 ml of chloroform. The chloroform solution is twice washed with 10 ml of water each. The organic solution is dried over anhydrous sodium sulfate and then evaporated. The final product is solidified by adding 50 ml of ether to obtain Z-MePhe-D-Trp-Phe-D-Trp-Leu $\psi$-(CH$_2$NH)Leu-NH$_2$ in a yield of 1.32 g (85 %), m.p.: 88 °C (decomposition); $[\alpha]_D{}^{20}$ = +5.7° (c = 1, methanol).

## Example 2

### Preparation of H-Leu-MPA

After suspending 26.7 g (60 mmol) of Z-Leu-OH di-cyclohexylamine salt in 300 ml of ethyl acetate, the salt is decomposed by 150 ml of 1 mol/l sulfuric acid solution. The ethyl acetate solution is dried over anhydrous sodium sulfate and evaporated. Z-Leu-OH obtained as a residue is dissolved in 160 ml of anhydrous tetrahydrofuran. After cooling the solution to -10 °C, 9.4 ml (67 mmol) of triethylamine are added, and then 9.0 ml (73 mmol) of pivaloyl chloride are dropwise added while maintaining the temperature at -10 °C. After stirring the reaction mixture at -10 °C for 15 minutes, a solution containing 6.7 g (74 mmol) of 2-amino-3-methylpentane in 20 ml of tetrahydrofuran are portionwise added. The reaction mixture is stirred at 0 °C for 30 minutes and at room temperature for 3 hours and then evaporated. The residue is dissolved in 300 ml of ethyl acetate and the solution is successively washed twice with 120 ml of 1 mol/l hydrochloric acid solution each, twice with 5 % sodium hydrogen carbonate solution and, finally, once with 120 ml of water. After drying and evaporation, Z-Leu-methylpentylamide is obtained as an oil in a yield of 20.5 g (98 %); $R_f{}^3$ = 0.8.

The obtained oily product is dissolved in 200 ml of methanol and hydrogenated in the presence of 2.5 g of 10 % palladium-on-carbon catalyst. The reaction becomes complete within 90 minutes. Subsequently, after filtering off the catalyst and evaporating methanol, the oily residue is separated into two components on a silica gel column (80x2.5 cm size; 250 g of silica gel; eluent: 6:1:3 mixture of ethyl acetate/methanol/n-hexane; flow rate: 40 ml/hour) and the fractions collected are evaporated. The yield of the thus obtained isomer of H-Leu-MPA is 4.2 g (19.5 mmol); $R_f^4$ = 0.3, $[\alpha]_D^{24}$ = +11.6° (c = 1, methanol); $[\alpha]_D^{24}$ = -20.4° (c = 1, ethyl acetate).

In the following Example, the isomer of Example 1 is used. Characteristics of a number of protected peptide amides and pseudopeptide amides are indicated in Table 5.

**Example 3**

### Preparation of D-MePhe-D-Trp-Phe-D-Trp-Leu $\psi(CH_2NH)$Leu-$NH_2$

A solution containing 1.0 g (0.96 mmol) of Z-D-MePhe-D-Trp-Phe-D-Trp-Leu $\psi(CH_2NH)$Leu-$NH_2$ in 25 ml of methanol is hydrogenated in the presence of 0.3 g of palladium-on-carbon catalyst for 3.5 hours. After filtering off the catalyst and evaporating the solution, the residue is solidified by adding ether to obtain the crude title product in a yield of 0.73 g.

The crude final product is purified on a silica gel column by using a 4:1 mixture of ethyl acetate with a 20:6:11 mixture of pyridine/acetic acid/water as eluent with a flow rate of 20 ml/hour. Fractions of 10 ml each are collected; the purity of the fractions is observed by thin layer chromatography. The fractions containing the pure final product are evaporated repeatedly twice with 10 ml of water each and twice with 10 ml of ethanol each. The water and ethanol are added to the evaporation reside in such a way that before adding an additional amount of liquid, the amount of liquid previously added is distilled off. The final evaporation residue is solidified by adding ether and after filtering, the precipitate is washed with ether to obtain the amorphous title product in a yield of 0.32 g; $[\alpha]_D^{20}$ = -22.8° (c = 0.5, 10 % acetic acid).

Amino acid analysis: Phe 1.0 (1), Trp 1.7 (2), MePhe, Leu $\psi(CH_2NH)$Leu.

Physical characteristics of the new compounds are summarized in Table 6.

## Table 5

| Compound | Yield (%) | M.p. (°C) | $[\alpha]_D^{20}$ (c=1, methanol) (°) |
|---|---|---|---|
| Z-D-Me-Phe-D-Trp-Phe-D-Trp--Leu $\psi$(CH$_2$NH)Leu-NH$_2$ | 75 | 120 (d) | +1.7 |
| Z-D-Me-Phe-D-Trp-Phe-D-Trp--Leu-MPA | 56 | 109 (d) | -22.9 |
| Boc-D-Tyr-D-Trp-Phe-D-Trp--Leu $\psi$(CH$_2$NH)Leu-NH$_2$ | 71 | 136 (d) | -19.2 |
| Boc-D-Tyr(Et)-D-Trp-Phe-D--Trp-Leu $\psi$(CH$_2$NH)Leu-NH$_2$ | 58 | 114 (d) | -23.4 |
| Z-D-Me-Phe-D-Trp-D-Tyr(Bzl)--D-Trp-Leu $\psi$(CH$_2$NH)Leu-NH$_2$ | 74 | 88 (d) | +5.7 |

Note: (d) means: decomposition.

11

## Table 6

| Compound | M.p. (°C) | $[\alpha]_D^{20}$ (°) |
|---|---|---|
| p-HOPA-D-Trp-Phe-D-Trp--Leu $\Psi$(CH$_2$NH)Leu-NH$_2$ | 132-137 | -29.1 (c=1, 50% AcOH) |
| D-Me-Phe-D-Trp-Phe-D-Trp--Leu $\Psi$(CH$_2$NH)Leu-NH$_2$ | 90 (d) | -22.8 (c=0.5, 10% AcOH) |
| D-Me-Phe-D-Trp-Phe-D-Trp--Leu MPA | 153-155 | -41.0 (c=1, EtOH) |
| D-Tyr-D-Trp-Phe-D-Trp--Leu $\Psi$(CH$_2$NH)Leu-NH$_2$ | 105 (d) | -17.1 (c=1, MeOH) |
| D-Tyr(Et)-D-Trp-Phe-D-Trp--Leu $\Psi$(CH$_2$NH)Leu-NH$_2$ | 166 (d) | -35.2 (c=1, MeOH) |
| D-Me-Phe-D-Trp-Tyr-D-Trp--Leu $\Psi$(CH$_2$NH)Leu-NH$_2$ | 110 (d) | -18.5 (c=1, 10% AcOH) |

Note: (d) means: decomposition.

### Example 4

Powder ampoule for injection purposes

500 mg. of active ingredient and 9.5 g. of lactose are dissolved in 80 ml. of distilled water suitable for injection purposes. 0.1 g. of methyl p-hydroxybenzoate are added to the solution, then the volume of this solution is adjusted to 100 ml. by using distilled water suitable for injection purposes. The homogenic solution is filtered to sterile, filled in a volume of 1 ml. each into vials fitted with gum cap, subjected to freeze-drying and finally the vials are provided with a gum plug. Powder ampoules containing 5 mg. of active ingredient each are obtained.

If one intends to obtain ampoules with a different active ingredient content, the amount of lactose is selected in such a manner that, based on 100 ml. of the solution, the common weight of the active ingredient and lactose is about 10 g. One can use mannitol in the same amount instead of lactose.

When administering an injection the powder of these ampoules is dissolved in an aqueous sodium chloride solution of such concentration which can provide an isotonic solution.

### Claims

1.  Peptide amides and pseudopeptide amides of formulae (1) to (6),

$$\text{p-HOPA-D-Trp-Phe-D-Trp-Leu } \Psi \text{ (CH}_2\text{NH)Leu-NH}_2 \qquad (1)$$

$$\text{D-MePhe-D-Trp-Phe-D-Trp-Leu } \Psi \text{(CH}_2\text{NH)Leu-NH}_2 \qquad (2)$$

$$\text{D-MePhe-D-Trp-Phe-D-Trp-Leu-MPA} \qquad (3)$$

$$\text{D-Tyr-D-Trp-Phe-D-Trp-Leu } \Psi \text{(CH}_2\text{NH)Leu-NH}_2 \qquad (4)$$

$$\text{D-Tyr(Et)-D-Trp-Phe-D-Trp-Leu } \Psi \text{(CH}_2\text{NH)Leu-NH}_2 \qquad (5)$$

$$\text{D-MePhe-D-Trp-Tyr-D-Trp-Leu}\ \psi(CH_2NH)\text{Leu-NH}_2 \qquad (6)$$

wherein

p-HOPA means p-hydroxyphenylacetic acid,

D-MePhe stands for D-N-methylphenylalanine,

MPA represents 2-amino-3-methylpentane and

$\psi(CH_2NH)$ is a methyleneamino group being present instead of a peptide bond,

and pharmaceutically acceptable acid addition salts thereof.

2. A process for the preparation of the peptide amides and pseudopeptide amides of formulae (1) to (6) as defined in Claim 1 which comprises using Leu(CH$_2$NH)Leu-NH$_2$ or Leu-MPA as a starting material, and carrying out successive reactive ester couplings and deprotection of any $\alpha$-NH$_2$ groups for each amino acid or other group to be added; followed if necessary by removal of any protective groups by catalytic hydrogenation or acidolysis, in order to produce a compound as defined in Claim 1.

3. A process as claimed in Claim 2 which comprises carrying out the reactive ester coupling step by using the pentafluorophenyl ester of the protected amino-acid to be added.

4. A process as claimed in Claim 2 or Claim 3 which comprises carrying out the step of reactive ester coupling by using a reactive ester derivative of the amino acid protected by a tert-butoxycarbonyl protective group.

5. A process as claimed in Claim 2 or Claim 3 which comprises carrying out the step of reactive ester coupling by using a reactive ester derivative of the amino acid protected by a benzyloxycarbonyl protective group.

6. A process as claimed in any one of Claims 2 to 4 which comprises removing the protective group by acidolysis.

7. A process as claimed in any one of Claims 2, 3 or 5 which comprises removing the protective group by catalytic hydrogenation.

8. A pharmaceutical composition which comprises as active ingredient a peptide amide or pseudopeptide amide of formulae (1) to (6) as defined in Claim 1 or a pharmaceutically acceptable acid addition salt thereof.

9. A process for the preparation of a pharmaceutical composition as defined in Claim 8 which comprises admixing the active ingredient as defined in Claim 8 with a pharmaceutically acceptable carrier, diluent or excipient.

European Patent Office

**EUROPEAN SEARCH REPORT**

Application Number

EP 93 30 0585

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | WO-A-8 807 551 (IMPERIAL CANCER RESEARCH TECHNOLOGY LIM.) 6 October 1988 --- | | C07K7/08 C07K15/00 A61K37/43 |
| A | BIOCHEMICAL AND BIOPHYSICAL RESEARCH COMMUNICATIONS vol. 156, no. 1, 14 October 1988, pages 323 - 327 P.SCHMITT ET AL. 'N-Acylated Pentapeptides Antagonists of Substance P an Guinea-Pig Ileum' ----- | | |

TECHNICAL FIELDS SEARCHED (Int. Cl.5)

C07K

The present search report has been drawn up for all claims

| Place of search | Date of completion of the search | Examiner |
|---|---|---|
| MUNICH | 14 MAY 1993 | DEFFNER C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)